# EUROPEAN PATENT SPECIFICATION

(11) **EP 2 976 049 B1**
(45) Date of publication and mention of the grant of the patent: **30.05.2018**
(21) Application number: 14710587.8
(22) Date of filing: 18.03.2014
(51) Int. Cl.: A61F 2/66

(54) **PROSTHETIC FOOT DEVICE**
FUSSPROTHESENVORRICHTUNG
DISPOSITIF DE PIED PROTHÉTIQUE

(30) Priority: 18.03.2013 SE 1350323
(43) Date of publication of application: 27.01.2016
(73) Proprietor: C LindheXtend AB, 302 50 Halmstad (SE)
(72) Inventor: LINDHE, Christoffer, 302 91 Halmstad (SE); KLINTÄNG, Anders, 302 48 Halmstad (SE)
(74) Representative: Valea AB
(86) International application number: PCT/EP2014/055411
(87) International publication number: WO 2014/147070

(56) References cited:
- WO-A1-97/33540
- US-A1- 2005 203 640
- US-A1- 2013 060 349
- US-B1- 6 406 500

## Description

### TECHNICAL FIELD

The invention relates generally to lower leg prostheses and, more particularly, to a lower prosthetic foot that simulates the performance characteristics of a natural foot.

### BACKGROUND OF THE INVENTION

A prosthetic foot is a very important constituent of a leg prosthesis. A prosthetic foot must reliably store and release energy while flexing in a number of degrees of motion so as to properly coordinate with the muscular action when the user is walking, running or standing. In addition it is desirable that the prosthetic foot is relatively inexpensive. Significant advancements have been made in the field during recent years, many of which are mechanically complex and employ a number of moving parts. While such devices provide good and reliable performance characteristics, their cost and complexity limit their use particular in high volume applications and in user communities, which do not have a sophisticated technical infrastructure to support and maintain such devices.

The development of composite materials technology, such as fiberglass/epoxy and carbon fiber/epoxy composite materials and their incorporation into prosthetic feet have improved the performance characteristics and feel of a prosthetic foot, resulting in an improved mobility for the wearer. However, few designs have focused on the lateral, or side to side rotation when the foot is used on varied or uneven terrain. The forefoot and heel of a natural foot rotates with a medial to lateral roll-over to accommodate variations in terrain. Many artificial feet of previous designs usually incorporate a unitary foot that is incapable of such lateral roll-over movement.

The object of the present invention is to provide a prosthetic foot which does not include any articulate spring members but which emulates containing articulate spring members, while still providing a comfortable and natural foot action. The prosthetic foot of the invention does not require any periodic maintenance or adjustment, and has relatively low production costs. Furthermore, due to the specific design of the prosthetic foot it offers an improved roll-over movement and stability when used on uneven terrain.

### SUMMARY OF THE INVENTION

This object may be achieved by a prosthetic foot device according to claim 1. The prosthetic foot device has a longitudinal extension from the heel region to the toe region corresponding to the longitudinal extension of a natural foot, and a lateral extension extending across the prosthetic device which is perpendicular to the longitudinal direction and corresponding to the width of a natural foot. The prosthetic foot device comprises an attachment unit configured to be connected to a coupling device, and/or to a stump of an amputee. The attachment unit is advantageously located in the ankle region or at a location above the ankle of a natural foot.

The prosthetic foot device further comprises a resilient upper forefoot member extending forwardly and downwardly from the attachment unit through the ankle region towards the toe region, said forefoot member comprising at least one elongated curvilinear forefoot leaf spring and at least one longitudinally extending parting slot dividing the toe region in at least two parts.

The device has a resilient heel member extending forwardly and downwardly from the attachment unit towards the toe region, curving through the ankle region and thereafter extending rearward towards the heel region, said heel member comprising at least one elongated curvilinear heel leaf spring. The prosthetic foot device also has a resilient lower foot member with a lower surface configured to engage the ground or surface for walking. The lower foot member is disposed below the forefoot and heel members of the prosthetic foot device. Generally the forefoot and heel members of the prosthetic foot device are located in the vertical direction above the lower foot plate when the lower surface of the lower foot plate engages a substantially horizontal ground in walking. Alternatively the lower surface of the lower foot member may engage the inside of a cosmesis or a shoe. The upper surface of the resilient lower foot member is attached to the forefoot member at said toe region and to the heel member in the heel region, and is laterally unitary along the longitudinal length thereof.

The resilient upper forefoot member is connected to, and extends from the attachment unit through the ankle region towards the toe region, said forefoot member comprising at least one elongated curvilinear forefoot leaf spring. The at least one curvilinear forefoot leaf spring is coupled to the attachment unit and terminates at a toe location corresponding to that of a natural foot. The attachment unit has an upper end configured to be connected to a coupling device, and/or to a stump of an amputee, and a bottom surface facing the upper forefoot member. Advantageously the bottom surface of the attachment unit is chamfered to coincide with the sloping angle of the upper forefoot member as this extends forwardly and downwardly from the attachment unit through the ankle region and towards the toe region. The upper end of the attachment unit is advantageously modifiable to adjust the angle between the prosthetic foot device and the coupling device, and/or to a stump of an amputee.

The prosthetic foot device contains a resilient heel member which extends from the attachment unit through the ankle region towards the heel region, said heel member comprising at least one elongated curvilinear heel leaf spring. Said at least one elongated curvilinear heel leaf spring may be connected to the forefoot member or coupled directly to the attachment unit. It extends downward and forwardly towards the toe region, curves around the ankle region and extends rearward towards the heel region located at a heel location of a natural foot.

The prosthetic foot device further contains a resilient lower foot member extending from a toe region to a heel region, located at a toe and heel location respectively of a natural foot. The resilient lower foot member is attached to the forefoot member at said toe region, and to the heel member at the heel region. The lower foot member is laterally unitary along the longitudinal length thereof, i.e. it contains no parting slots. The resilient lower foot member extends a longitudinal length corresponding to that of a natural foot, and can be essentially flat and horizontal. However, the lower foot member may have curvatures including a downward curvature or valley in the area of a ball and heel location, and upward curvatures or peaks at the toe and arch locations of a natural foot.

The forefoot and heel leaf springs, as well as the lower foot member mentioned above, are made from a material that permit them to bend and deflect under force to store energy, and thereafter return to their original shape releasing energy as the force is removed. The leaf springs are advantageously flexible and resilient, and can be a composite, and include fibers, such as graphite fibers in a resin matrix. The springs can be straight or flat in the lateral, or side to side direction, while being curved in the longitudinal, or toe to heel direction.

The at least one leaf spring of the upper forefoot member has at least one longitudinally extending parting slot dividing the toe region in at least two parts. Advantageously, the at least one forefoot member contains one longitudinally extending parting slot, dividing the toe region into two parts. Thus, the at least one forefoot leaf spring of the forefoot member is vertically bifurcated (i.e. It has a cut through the leaf spring) extending from the toe end towards the attachment unit. The parting slot extends along the longitudinal length of the forefoot member, dividing the toe region in substantially two or more equal parts in the lateral direction. In one embodiment of the invention the parting slot may be located a bit off-center, i.e. the parting slot divides the toe region into two unequal parts in the lateral direction. Advantageously the part corresponding to the inside part of a natural foot is smaller, thereby simulating a big toe of a natural foot. In one embodiment the at least one forefoot member contains two longitudinally extending parting slots, dividing the toe region into three parts. In one further embodiment the at least one forefoot member contains three longitudinally extending parting slots, dividing the toe region into four parts.

The one or more parting slots allow the toe region to flex more independently, more like a natural foot and permit the foot to respond to uneven terrain. However, it is important that the at least one leaf spring having the at least one parting slot is connected to a lower foot member which is unitary in the toe region, i.e. it contains no parting slots. The advantages of having a unitary lower foot member (i.e. a leaf spring without any parting slot), compared to one comprising parting slots are many. With a unitary lower foot member as the one in the foot prosthetic device of the invention, the foot prosthesis device is endowed with a resilience which is more evenly distributed across the entire forefoot during load when the prosthesis is biased.

Furthermore, with a unitary lower foot member, the foot prosthetic device becomes more stable without having to increase the thickness of any of the leaf springs. If both the forefoot leaf spring and the lower foot member are bifurcated, the leaf springs must be thicker to provide the required stability to the foot prosthesis. An increased thickness of the leaf springs leads to a foot prosthesis with an overall increased stiffness and less resiliency. This was proven in a trial, wherein a person of 60 kg, when walking on a smooth terrain, stepped on a small obstacle 1.5 cm high. When wearing a prosthetic foot wherein both the forefoot member and the lower foot member contained a parting slot, the prosthetic foot adjusted 7° when the wearer stepped on the obstacle. This should be compared to an adjustment of 14-15°, when wearing the prosthetic foot of the invention.

The lower foot member is connected to the forefoot member and the heel member by elastomeric adhesive having a Young's modulus (E) of less than 13 MPa. As used herein the term Young's modulus (also known as tensile modulus) is intended to mean the measure of the stiffness of an elastic material, such as the elastomeric adhesive that is used to connect the lower foot member to the forefoot and heel members. The Young's modulus calculates the change in the dimension of a piece made of an isotropic elastic material under tensile or compressive loads. For instance, it predicts how much a material sample, e.g. the adhesive, extends under tension or shortens under compression. The Young' modulus is expressed in units of pressure, pascal (Pa or N/m² or m⁻¹ x kg x s⁻²), but the more practical unit when used in this invention is megapascal (Mpa or N/mm²).

A stiff material has a high Young's modulus and changes its shape only slightly under elastic loads (e.g. diamond). A flexible material has a low Young's modulus and changes its shape considerably (e.g. rubber). The elastomeric adhesive used to connect the lower foot member to the forefoot and heel members should be flexible enough to provide the foot with resiliency and flexibility, yet stiff enough to withstand the load of a wearer during regular use, such as standing, walking, running and jumping. Advantageously, the elastomeric adhesive used to connect the lower foot member to the forefoot and heel members should have a Young's modulus (E) of less than 13 MPa.

The at least one parting slot has a length that extends from an anterior toe region along only 15-50%, more preferably only 25-35 %, most preferably about only 30 % of the length of the forefoot member. This means that less than half and more preferably around one third of the forefoot member in the longitudinal direction contains the one or more parting slots. The elastomeric adhesive is applied to first bonding areas between the forefoot member and the lower foot member, said first bonding areas extending in the longitudinal direction from the anterior toe region along 25-60%, more preferably along 30-55%, most preferably 40-50% of the length of the at least one parting slot of the forefoot member and towards the ankle region, and 30-60 %, preferably 40-55%, and most preferably 50 % of the width in the lateral directions of said foot prosthesis from said parting slot.

This means that the elastomeric adhesive is applied in only part of a possible first contact area, i.e. the area available for contact between the forefoot member and the lower foot member. Said first contact area covers the area between the forefoot member and the lower foot member, extending from side to side in the lateral direction and from the anterior toe region and in the longitudinal direction of the foot until the forefoot member curves and deflects upwards away from the lower foot member. Said first bonding areas between said forefoot and lower foot members extend from the anterior toe region, i.e. the foremost end of the toe region and in the longitudinal direction of the forefoot member towards the ankle region along 25-60%, more preferably along 30-55%, most preferably 40-50% of the length on each side of the at least one parting slot, and in the lateral direction starting from both sides of the one or more parting slots covering 30-60 %, preferably 40-55%, and most preferably 50 % of the width in said lateral directions. This means that the first bonding areas generally cover the areas extending from the anterior toe region and close to each side of the parting slot. The outermost areas between the lower foot member and the forefoot members in the lateral directions from the parting slot are free from elastomeric adhesive, allowing the forefoot member and lower foot member to move freely with respect to each other in the toe region. However, it is important that no elastomeric adhesive is applied to the parting slot itself.

The elastomeric adhesive is applied to a second bonding area between the forefoot member and the lower foot member in the heel region.

The forefoot member may comprise at least two elongated curvilinear forefoot leaf springs connected in the toe region, forming furcated forwardly slightly concave arcs, that may define gap between the at least two forefoot leaf springs in the ankle region. Advantageously, the forefoot member comprises two forefoot leaf springs forming vertically bifurcated forwardly concave arcs which define a gap between said two forefoot leaf springs in the ankle region. The gap between the at least two forefoot leaf springs endows said forefoot member with an improved resiliency, as the forefoot leaf springs, independently from each other, deflect under force to store energy, and return to their original shape releasing energy as the force is removed, thereby improving the overall resiliency of the foot prosthesis. Advantageously there is a damper located between each of the at least two forefoot leaf springs at the location where the forefoot leaf springs are attached to the attachment unit. The dampers will aid in providing a gap between said forefoot leaf springs.

The forefoot leaf springs of the forefoot member are connected by elastomeric adhesive applied to third bonding areas, said third bonding areas extending in the longitudinal direction from the anterior toe region along 25-60%, more preferably along 35-55%, most preferably 40-50% of the length of the parting slots of the forefoot leaf springs and towards the ankle region, and at least 50 %, preferably at least 75 %, and most preferably at least 90 % of the width in the lateral directions from said parting slots on the forefoot leaf springs.

This means that the elastomeric adhesive is applied in only part of a possible third contact area. Said third contact area covers the area available for contact between the forefoot leaf springs, i.e. the entire toe region between the forefoot leaf springs, extending from one side to the other side of the forefoot leaf springs in the lateral direction, and from the anterior toe region in the longitudinal direction of the foot until the forefoot leaf springs curve and deflect away from teach other in the ankle region. Said third bonding areas between the forefoot leaf springs extend from the anterior toe region, i.e. the foremost end of the toe region and in the longitudinal direction of the forefoot leaf springs towards the ankle region, along 25-60%, more preferably along 35-55%, most preferably 40-50% of the length on each side of the at least one parting slot, and in the lateral direction starting from both sides of the one or more the parting slots, covering at least 50 %, preferably at least 75 %, and most preferably at least 90 % of the width in said lateral directions. This means that the third bonding area generally covers the area extending from the anterior toe region and adjacent to both sides of the parting slot. The outermost areas in the lateral direction between the two forefoot leaf springs are free from elastomeric adhesive, allowing the forefoot leaf springs to move freely with respect to each other along the outer edges in the toe region. However, it is important that no elastomeric adhesive is applied to the parting slot itself.

The heel member may comprise at least two elongated curvilinear heel leaf springs which extend from the attachment unit downwardly and forwardly towards the toe region, curve around the ankle region and thereafter extend rearward towards the heel region.. Advantageously the heel member comprises two elongated curvilinear heel leaf springs connected in the heel region defining a gap between said heel leaf springs in the ankle region. The gap between the at least two forefoot leaf springs endows said heel member with an improved resiliency as the leaf springs, independently from one another, deflect under force to store energy, and return to their original shape releasing energy as the force is removed.Said heel leaf springs are connected to each other by elastomeric adhesive applied to a fourth bonding area.

The elastomeric adhesive applied to the first and second bonding areas may be a first elastomeric adhesive having a lower tensile modulus (E) than a second elastomeric adhesive applied to the third and fourth bonding areas. Advantageously, elastomeric adhesives having different tensile moduli are used in the different bonding areas to improve the resiliency and flexibility of the prosthetic foot during gait.

Using an elastomeric adhesive with higher tensile modulus will improve the stiffness and also the durability of the bonding regions, but will at the same time make the bond less resilient. An elastomeric adhesive with a low tensile modulus will improve the resiliency considerably, but decrease the durability of the bonding region. Thus, the use of elastomeric adhesives in the present invention is a delicate balance between providing a prosthetic foot which has maximum resiliency for optimal gait, yet which is stiff enough to provide the durability required for a foot which is constantly exposed to strain. Advantageously the first elastomeric adhesive has a tensile modulus (E) of less than 5 MPa, more preferably, less than 3 MPa, most preferably about 1 MPa, and advantageously the second elastomeric adhesive has a tensile modulus (E) of 13 -7 MPa, more preferably, 12-8 MPa, most preferably about 10 MPa.

The forefoot leaf springs, heel leaf springs and the lower foot member are made from a flexible and resilient material, such as a composite material with fibres in a resin matrix. Advantageously the flexible and resilient material is carbon fibres or fiberglass.

The specific design of the prosthetic foot device allows the at least two forefoot spring leafs of the forefoot member and the lower foot member to cooperate during gait and thereby create a stable, yet at the same time a flexible prosthesis foot with an improved adaptability to the terrain. When a prosthesis foot made from less flexible or thicker spring leafs are biased due to an uneven ground or a pebble, it is too stiff to adapt to the obstacle, and a levering action will transplant itself through the body trying to reposition the body sidewise in order for the foot to establish contact with the ground. The levering action will make the user try to cancel the levering force, which has the result that he will end up standing on the top of the obstacle instead.

Thin spring leafs, together with the bifurcated toe region of the forefoot member being attached to a unitary lower foot member as well as the use of elastomeric adhesives with different tensile modulus attaching said lower foot member to the forefoot and heel members, will work together and form a prosthetic foot that offers an improved roll-over movement and stability when used on uneven terrain.

### BRIEF DESCRIPTION OF THE DRAWINGS

Figure 1 is a perspective view of an embodiment of the prosthetic foot device of the invention
Figure 2 is a side view of the prosthetic foot device of Fig. 1
Figure 3 is a bottom view of the prosthetic foot device of Fig. 1
Figure 4 is an exploded view of the prosthetic foot device of Fig. 1
Figure 5 is a perspective view of a further embodiment of the prosthetic foot device of the invention.
Figure 6 is a top view of the prosthetic device of Fig. 5.

### DETAILED DESCRIPTION OF PREFERRED EMBODIMENTS

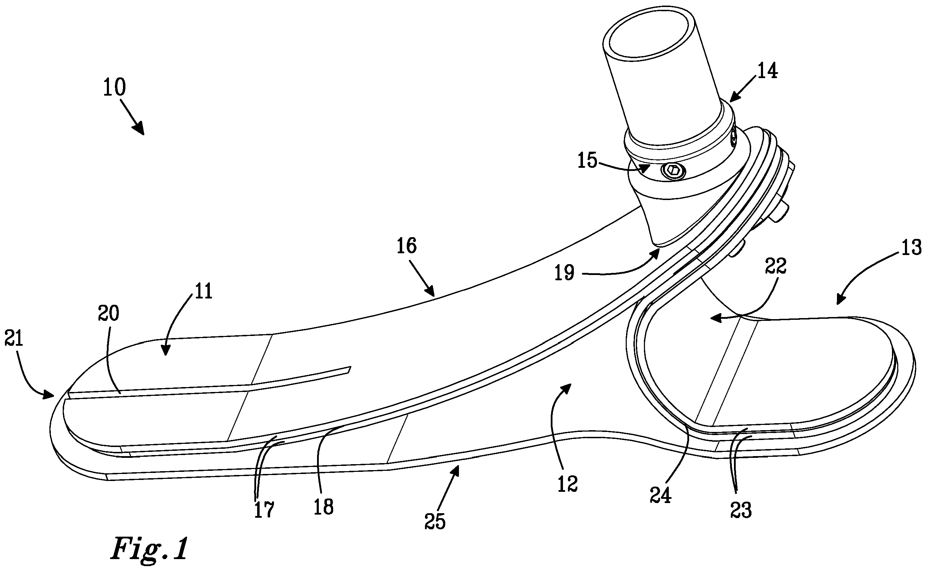

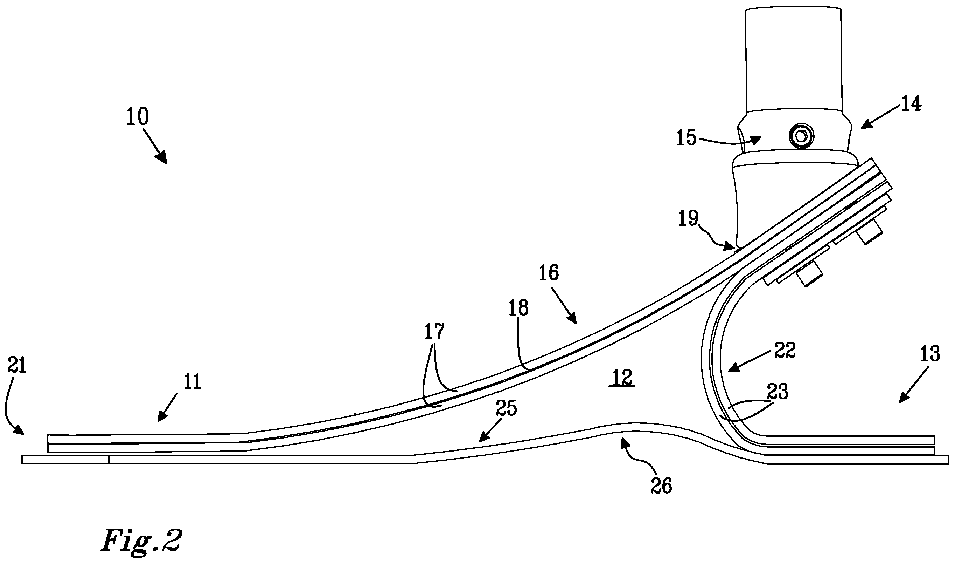

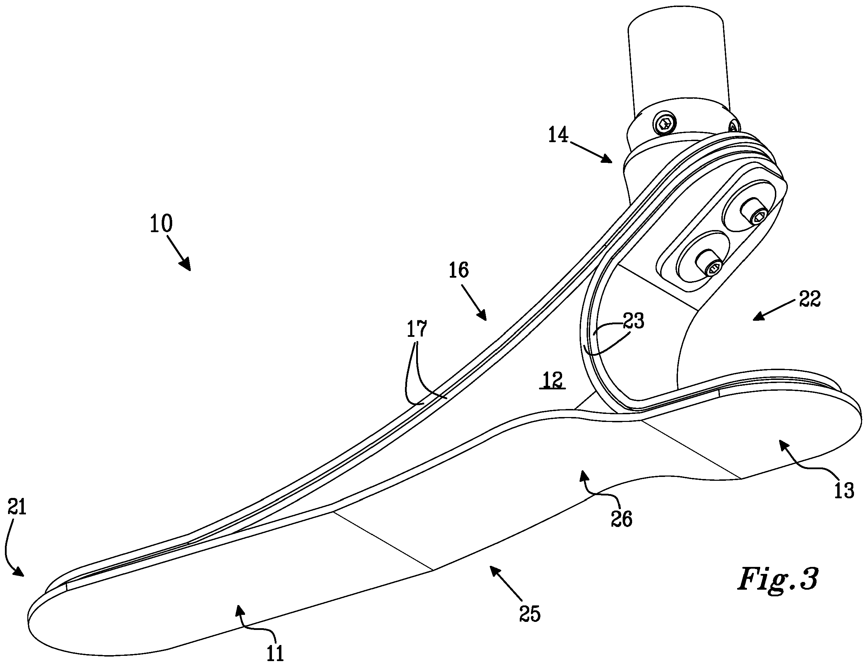

In the following the invention will be described in a non-limiting way and in more detail with reference to exemplary embodiments illustrated in the enclosed drawings 1-4, in which figures 1-3 show a schematic view of the prosthesis foot 10 of the invention, as seen from different angles. The prosthetic foot device 10 of the present invention has a toe region 11, an ankle region 12 and a heel region 13, corresponding to a toe location, an ankle location, and a heel location respectively of a natural foot. The prosthetic foot device 10 comprises an attachment unit 14 configured to be connected to coupling device (not shown) and/or to a stump of an amputee. The upper end of the attachment unit 15 may be adjustable to modify the angle between the prosthetic foot 10 and the leg of the amputee (not shown).

A resilient upper forefoot member 16 comprising at least one elongated curvilinear forefoot leaf spring 17 is connected to, and extends from the attachment unit 14 through the ankle region 12 towards the toe region 11. Advantageously,the forefoot member 16 comprises at least two elongated curvilinear forefoot leaf springs 17 connected in the toe region 11, forming two slightly concave arcs, defining a first gap 18 between the two forefoot leaf springs 17 in the ankle region 12.

In this embodiment the two curvilinear forefoot leaf springs 17 are coupled to the attachment unit 14, extend forwardly and downwardly and terminate at the region of a toe location of a natural foot. The bottom surface of the attachment unit 19 is chamfered to coincide with the same sloping angle as the upper forefoot member 16. A damper (not shown) may be arranged between the two forefoot leaf springs 17 at the attachment unit 14 to provide a gap 18 between said forefoot leaf springs 17, as well as preventing any rubbing of said leaf springs against each other, at the connection with the attachment unit 14.

The least one forefoot leaf spring 17 of the upper forefoot member 16 has at least one longitudinally extending parting slot 20 dividing the toe region 11 in at least two parts. Advantageously the upper forefoot member 16 has one parting slot 20 as seen in figures 1-4. The parting slot 20 has a length that extends from the anterior toe region 21 along only 15-40%, more preferably only 25-35 %, most preferably only 30 % of the length of the forefoot member 16. This means that less than half and more preferably around one third of the forefoot member 16 in the longitudinal direction contains a parting slot 20.

The prosthetic foot device 10 contains a resilient heel member 22 comprising at least one elongated curvilinear heel leaf spring 23, connected to and extending from the attachment unit 14 through the ankle region 12 towards the heel region. Figures 1-4 disclose a heel member 22 of a prosthetic foot with two elongated curvilinear heel leaf springs 23 which extend from the attachment unit 14 downward and forwardly towards the toe region, curve around the ankle region 12 and rearward towards the heel region 13. A second gap 24 is formed between the two heel leaf springs 23 in the ankle region 12. A damper (not shown) may be arranged between the two heel leaf springs 23 at the attachment unit 14 to provide a gap between said heel leaf springs 23, as well as preventing any rubbing of said leaf springs against each other, at the connection with the attachment unit 14.

The prosthetic foot device 10 further contains a resilient and laterally unitary lower foot member 25 extending from a toe region 11 to a heel region 13, located at a toe and heel location respectively of a natural foot. The resilient lower foot member 25 is attached to the forefoot member 16 in said toe region 11, and to the heel member 22 in the heel region 13. The resilient lower foot member 25 extends a longitudinal length of a foot, and is essentially flat and horizontal, but may have curvatures including a downward curvature or valley 26 in the area of a ball and heel location, and upward curvatures or peaks 27 at the toe and arch locations of a natural foot.

The lower foot member 25 is connected to the forefoot member 16 and the heel member 22 by means of elastomeric adhesive having a Young's modulus (E) of less than 13 MPa.

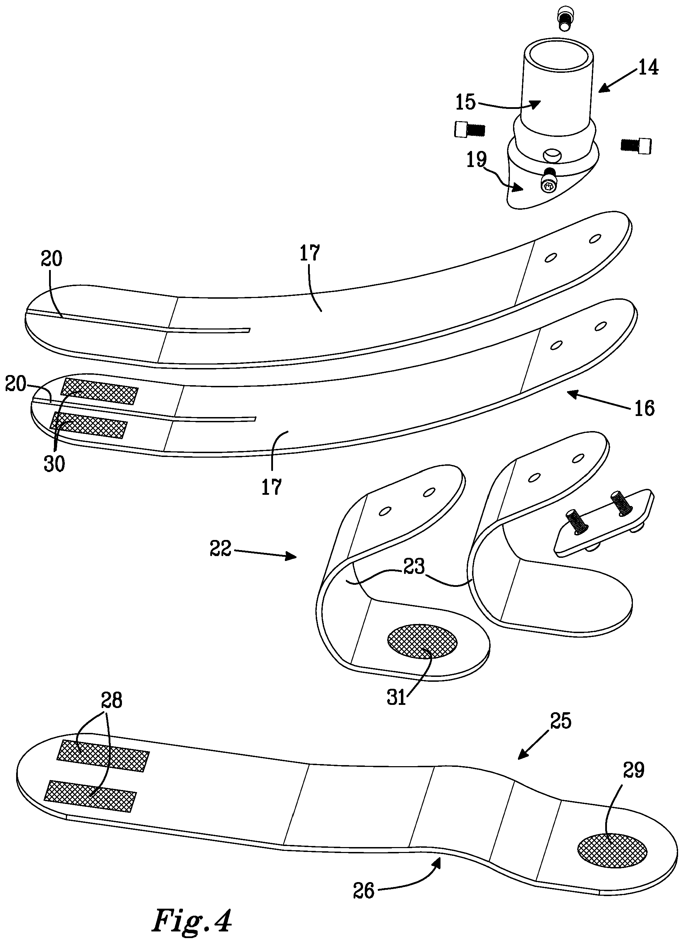

The elastomeric adhesive is applied to first bonding areas 28 between the forefoot member 16 and the lower foot member 25. As can be seen in figure 4, said first bonding areas 28 extend in the longitudinal direction from the anterior toe region 20 along 25-60%, more preferably along 35-55%, most preferably 40-50% of the length of the at least one parting slot 20 of the forefoot member 16 and towards the ankle region 12, and 30-60 %, preferably 40-55%, and most preferably 50 % of the width in the lateral directions of said foot prosthesis from said parting slot 20. The outermost areas between the lower foot member 25 and the forefoot member 16 in the lateral directions from the parting slot 20 are free from elastomeric adhesive, allowing the forefoot member 16 and lower foot member 25 to move freely with respect to each other in the toe region 11. However, it is important that no elastomeric adhesive is applied to the parting slot 20 itself. The elastomeric adhesive is applied to a second bonding area 29 between the forefoot member 16 and the lower foot member in the heel region 13.

The forefoot leaf springs 17 of the forefoot member 16 are connected by elastomeric adhesive applied to third bonding areas 30. As can be seen in figure 4, said third bonding areas 30 extend in the longitudinal direction from the anterior toe region 20 along 25-60%, more preferably along 35-55%, most preferably 40-50% of the length of the parting slots 20 of the forefoot leaf springs 17 and towards the ankle region 12, and at least 50 %, preferably at least 75 %, and most preferably at least 90 % of the width in the lateral directions from said parting slots 20 on the forefoot leaf springs 17. The heel leaf springs 23 are connected to each other by elastomeric adhesive applied to a fourth bonding area 31.

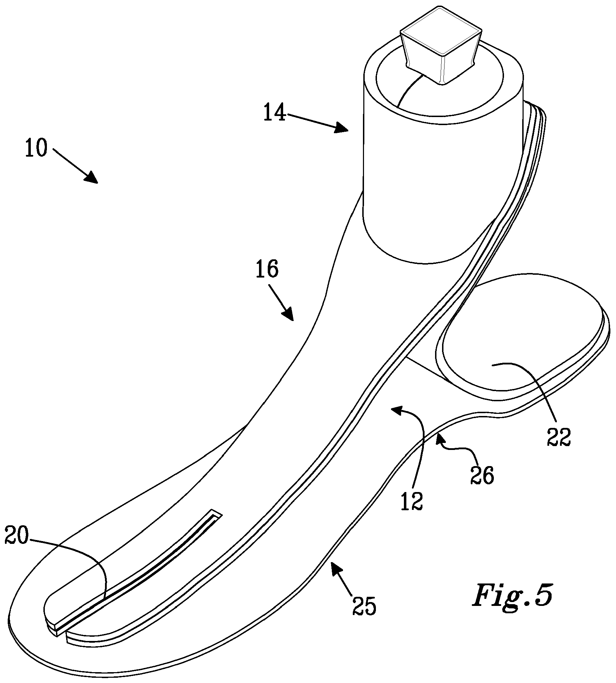

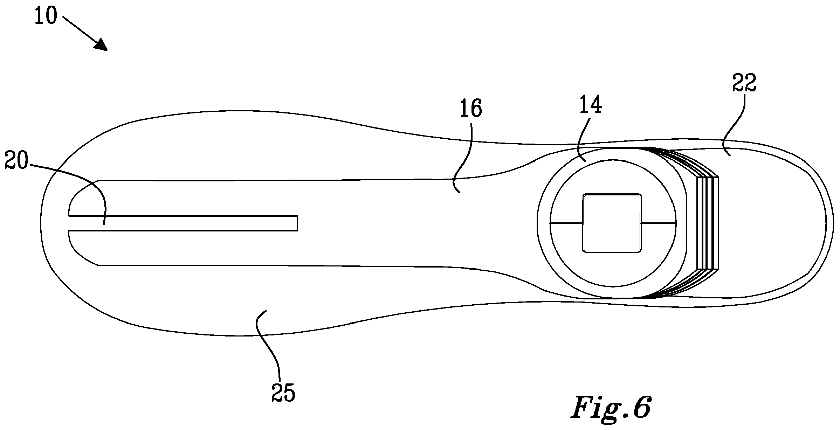

Figures 5 and 6 disclose a further embodiment of the prosthetic foot device 10. In this embodiment the forefoot member 16 and the heel member 22 are narrower than the lower foot member 25. The narrower shapes of the forefoot member 16 and in particular the heel member 22 provide the prosthetic device with an enhanced ability to bend or twist sideways during gait. Furthermore, the lower foot member 25 is provided with a "waist", i.e. a narrowing of the foot member 25 in the lateral direction, located in between the toe region and the heel region of the lower foot member 25. This "waist" enables the prosthetic foot device to better adjust to any unevenness on the ground.

## Claims

1. A prosthetic foot device (10) having a toe region (11), an ankle region (12) and a heel region (13), said device comprising
- an attachment unit (14) configured to be connected to a coupling device and/or to a stump of an amputee;
- an upper forefoot member (16) extending from the attachment unit (14) through the ankle region (12) towards the toe region (11), said forefoot member (16) comprising at least one elongated curvilinear forefoot leaf spring (17) and at least one longitudinally extending parting slot (20) dividing the toe region (11) of the fore foot member (16) in at least two parts:
- a heel member (22) extending from the attachment unit (14) through the ankle region (12) towards the heel region (13), said heel member (22) comprising at least one elongated curvilinear heel leaf spring (23);
- a lower foot member (25) attached to said heel member (22) in the heel region (13), **characterized in that** the lower foot member (25) is attached to the forefoot member (16) at said toe region (11), and that the lower foot member (25) is laterally unitary along the longitudinal length thereof and **in that** the at least one parting slot (20) has a length extending from an anterior toe region (21) towards the attachment unit (14) along only 15-50% of the longitudinal length of the forefoot member (16).

2. The prosthetic foot device (10) according to claim 1, wherein the at least one parting slot (20) has a length extending from an anterior toe region (21) towards the attachment unit (14) along only 25-35 %, most preferably along only 30 % of the longitudinal length of the forefoot member (16).

3. The prosthetic foot device (10) according to claims 1-2, wherein the lower foot member (25) is connected to the forefoot member (16) and the heel member (22) by an elastomeric adhesive having a tensile modulus (E) of less than 13 MPa.

4. The prosthetic foot device (10) according to claim 3, wherein the elastomeric adhesive is applied to first bonding areas (28) between the forefoot member (16) and the lower foot member (25) in the toe region (11), said first bonding areas (28) extending in the longitudinal direction from the anterior toe region (11) along 25-60%, more preferably along 30-55%, most preferably 40-50% of the length of the at least one parting slot (20) of the forefoot member (16) and towards the ankle region (12), and 30-60 %, preferably 40-55%, and most preferably 50 % of the width in the lateral directions from said parting slot (20).

5. The prosthetic foot device (10) according to claims 2-4, wherein the elastomeric adhesive is applied to a second bonding area (29) between the forefoot member (16) and the lower foot member (25) in the heel region (13).

6. The prosthetic foot device (10) according to any one of the preceding claims, wherein the forefoot member (16) comprises at least two elongated curvilinear forefoot leaf springs (17) being connected in the toe region (11) and forming furcated and forwardly concave arcs defining first gaps (18) between the at least two forefoot leaf springs (17) in the ankle region (12).

7. The prosthetic foot device (10) according to any one of the preceding claims, wherein theforefoot member (16) comprises two elongated curvilinear forefoot leaf springs (17) being connected in the toe region (11) and forming bifurcated forwardly concave arcs defining a first gap (18) between said forefoot leaf springs (17) in the ankle region (12).

8. The prosthetic foot device (10) according to anyone of claims 6-7, wherein the forefoot leaf springs (17) of the upper forefoot member (16) are connected by elastomeric adhesive applied to third bonding areas (30), said third bonding areas (30) extending in the longitudinal direction from the anterior toe region (11) along 25-60%, more preferably along 35-55%, most preferably along 40-50% of the length of the at least one parting slot (20) of the forefoot leaf springs (17) and towards the ankle region (12), and at least 50 %, preferably at least 75 %, and most preferably at least 90 % of the length in the lateral directions from said parting slot (20).

9. The prosthetic foot device (10) according to anyone of claims 1-8, wherein the heel member (22) comprises at least two elongated curvilinear heel leaf springs (23) extending from the attachment unit (14) downward and forwardly towards the toe region, curve around the ankle region (12) and rearward towards the heel region (13) defining second gaps (24) between the at least two heel leaf springs (23) in the ankle region (12).

10. The prosthetic foot device (10) according to anyone of claims 1-9, wherein the heel member (22) comprises two elongated curvilinear heel leaf springs (23) extending from the attachment unit (14) downward and forwardly towards the toe region, curve around the ankle region (12) and rearward towards the heel region (13) defining a second gap (24) between the two heel leaf springs (23) in the ankle region (12).

11. The prosthetic foot device (10) according to anyone of claims 9-10, wherein heel leaf springs (23) are connected by elastomeric adhesive applied to a fourth bonding area (31).

12. The prosthetic foot device (10) according to any one of the preceding claims 4-11, wherein the elastomeric adhesive applied to the first (28) and second bonding (29) areas is a first elastomeric adhesive having a lower tensile modulus (E) than a second elastomeric adhesive applied to the third (30) and fourth bonding (31) areas.

13. The prosthetic foot device (10) according to claim 12, wherein the first elastomeric adhesive has a tensile modulus (E) of less than 5 MPa, more preferably, less than 3 MPa, most preferably 1 MPa.

14. The prosthetic foot device (10) according to claim 12, wherein the second elastomeric adhesive has a tensile modulus (E) of 13 -7 MPa, more preferably, 12-8 MPa, most preferably 10 MPa.

15. The prosthetic foot device (10) according to anyone of the preceding claims 1-14, wherein the forefoot leaf springs (17), heel leaf springs (23) and the lower foot member (25) are made from a flexible and resilient material.

## Patentansprüche

1. Fußprothesenvorrichtung (10) mit einem Zehenbereich (11), einem Knöchelbereich (12) und einem Fersenbereich (13), wobei die Vorrichtung umfasst:
- eine Befestigungseinheit (14), die konfiguriert ist, um mit einer Kupplungsvorrichtung und/oder mit einem Stumpf eines Amputierten verbunden zu werden;
- ein oberes Vorfußelement (16), das sich von der Befestigungseinheit (14) durch den Knöchelbereich (12) in Richtung des Zehenbereichs (11) erstreckt, wobei das Vorfußelement (16) zumindest eine längliche gekrümmte Vorfuß-Blattfeder (17) und zumindest einen sich longitudinal erstreckenden Trennschlitz (20) umfasst, der den Zehenbereich (11) des Vorfußelements (16) in zumindest zwei Teile teilt:
- ein Fersenelement (22), das sich von der Befestigungseinheit (14) durch den Knöchelbereich (12) in Richtung des Fersenbereichs (13) erstreckt, wobei das Fersenelement (22) zumindest eine längliche gekrümmte Fersen-Blattfeder (23) umfasst;
- ein unteres Fußelement (25), das an dem Fersenelement (22) in dem Fersenbereich (13) befestigt ist, **dadurch gekennzeichnet, dass**
das untere Fußelement (25) an dem Vorfußelement (16) in dem Zehenbereich (11) befestigt ist und dass
das untere Fußelement (25) seitlich einheitlich entlang der longitudinalen Länge davon ist und dass der zumindest eine Trennschlitz (20) eine Länge hat, die sich von einem vorderen Zehenbereich (21) in Richtung der Befestigungseinheit (14) entlang nur 15-50% von der longitudinalen Länge des Vorfußelements (16) erstreckt.

2. Fußprothesenvorrichtung (10) nach Anspruch 1, wobei der zumindest eine Trennschlitz (20) eine Länge hat, die sich von einem vorderen Zehenbereich (21) in Richtung der Befestigungseinheit (14) entlang nur 25-35%, besonders bevorzugt entlang nur 30% von der longitudinalen Länge des Vorfußelements (16) erstreckt.

3. Fußprothesenvorrichtung (10) nach den Ansprüchen 1-2, wobei das untere Fußelement (25) mit dem Vorfußelement (16) und dem Fersenelement (22) durch einen elastomeren Klebstoff mit einem Elastizitätsmodul (E) von weniger als 13 MPa verbunden ist.

4. Fußprothesenvorrichtung (10) nach Anspruch 3, wobei der elastomere Klebstoff auf erste Verklebungsbereiche (28) zwischen dem Vorfußelement (16) und dem unteren Fußelement (25) in dem Zehenbereich (11) aufgebracht ist, wobei sich die ersten Verklebungsbereiche (28) in der Längsrichtung von dem vorderen Zehenbereich (11) entlang 25-60%, bevorzugter entlang 30-55%, besonders bevorzugt 40-50% von der Länge des zumindest einen Trennschlitzes (20) des Vorfußelements (16) und in Richtung des Knöchelbereichs (12) und 30-60%, bevorzugt 40-55% und besonders bevorzugt 50% von der Breite in den seitlichen Richtungen von dem Trennschlitz (20) erstrecken.

5. Fußprothesenvorrichtung (10) nach den Ansprüchen 2-4, wobei der elastomere Klebstoff auf einen zweiten Verklebungsbereich (29) zwischen dem Vorfußelement (16) und dem unteren Fußelement (25) in dem Fersenbereich (13) aufgebracht ist.

6. Fußprothesenvorrichtung (10) nach einem der vorstehenden Ansprüche, wobei das Vorfußelement (16) zumindest zwei längliche gekrümmte Vorfuß-Blattfedern (17) umfasst, die in dem Zehenbereich (11) verbunden sind und gegabelte und nach vorne konkave Bögen bilden, die erste Spalte (18) zwischen den zumindest zwei Vorfuß-Blattfedern (17) in dem Knöchelbereich (12) definieren.

7. Fußprothesenvorrichtung (10) nach einem der vorstehenden Ansprüche, wobei das Vorfußelement (16) zwei längliche gekrümmte Vorfuß-Blattfedern (17) umfasst, die in dem Zehenbereich (11) verbunden sind und gegabelte nach vorne konkaven Bögen bilden, die einen ersten Spalt (18) zwischen den Vorfuß-Blattfedern (17) in dem Knöchelbereich (12) definieren.

8. Fußprothesenvorrichtung (10) nach einem der Ansprüche 6-7, wobei die Vorfuß-Blattfedern (17) des oberen Vorfußelements (16) durch elastomeren Klebstoff verbunden sind, der auf dritte Verklebungsbereiche (30) aufgebracht ist, wobei sich die dritten Verklebungsbereiche (30) in der Längsrichtung von dem vorderen Zehenbereich (11) entlang 25-60%, bevorzugter entlang 35-55%, besonders bevorzugt entlang 40-50% von der Länge des zumindest einen Trennschlitzes (20) der Vorfuß-Blattfedern (17) und in Richtung des Knöchelbereichs (12) und zumindest 50%, bevorzugt zumindest 75% und besonders bevorzugt zumindest 90% von der Länge in den seitlichen Richtungen von dem Trennschlitz (20) erstrecken.

9. Fußprothesenvorrichtung (10) nach einem der Ansprüche 1-8, wobei das Fersenelement (22) zumindest zwei längliche gekrümmte Fersen-Blattfedern (23) umfasst, die sich von der Befestigungseinheit (14) nach unten und nach vorne in Richtung des Zehenbereichs erstrecken, die sich um den Knöchelbereich (12) herum und nach hinten in Richtung des Fersenbereichs (13) biegen, die zweite Spalte (24) zwischen den zumindest zwei Fersen-Blattfedern (23) in dem Knöchelbereich (12) definieren.

10. Fußprothesenvorrichtung (10) nach einem der Ansprüche 1-9, wobei das Fersenelement (22) zwei längliche gekrümmte Fersen-Blattfedern (23) umfasst, die sich von der Befestigungseinheit (14) nach unten und nach vorne in Richtung des Zehenbereichs erstrecken, die sich um den Knöchelbereich (12) herum und nach hinten in Richtung des Fersenbereichs (13) biegen, die einen zweiten Spalt (24) zwischen den zwei Fersen-Blattfedern (23) in dem Knöchelbereich (12) definieren.

11. Fußprothesenvorrichtung (10) nach einem der Ansprüche 9-10, wobei Fersen-Blattfedern (23) durch elastomeren Klebstoff verbunden sind, der auf einen vierten Verklebungsbereich (31) aufgebracht ist.

12. Fußprothesenvorrichtung (10) nach einem der vorstehenden Ansprüche 4-11, wobei der elastomere Klebstoff, der auf die ersten (28) und zweiten Verklebungs-(29)-bereiche aufgebracht ist, ein erster elastomerer Klebstoff mit einem niedrigeren Elastizitätsmodul (E) als ein zweiter elastomerer Klebstoff, der auf die dritten (30) und vierten Verklebungs-(31)-bereiche aufgebracht ist, ist.

13. Fußprothesenvorrichtung (10) nach Anspruch 12, wobei der erste elastomere Klebstoff ein Elastizitätsmodul (E) von weniger als 5 MPa, bevorzugter weniger als 3 MPa, besonders bevorzugt 1 MPa hat.

14. Fußprothesenvorrichtung (10) nach Anspruch 12, wobei der zweite elastomere Klebstoff ein Elastizitätsmodul (E) von 13-7 MPa, bevorzugter 12-8 MPa, besonders bevorzugt 10 MPa hat.

15. Fußprothesenvorrichtung (10) nach einem der vorstehenden Ansprüche 1-14, wobei die Vorfuß-Blattfedern (17), die Fersen-Blattfedern (23) und das untere Fußelement (25) aus einem flexiblen und robusten Material hergestellt sind.

## Revendications

1. Dispositif de pied prothétique (10) ayant une région d'orteil (11), une région de cheville (12) et une région de talon (13), ledit dispositif comprenant
- une unité de fixation (14) configurée pour être reliée à un dispositif de couplage et/ou à un moignon d'un amputé ;
- un membre d'avant-pied supérieur (16) s'étendant de l'unité de fixation (14) à travers la région de cheville (12) vers la région d'orteil (11), ledit membre d'avant-pied (16) comprenant au moins un ressort à lames d'avant-pied curvilinéaire allongé (17) et au moins une rainure de séparation s'étendant longitudinalement (20) divisant la région d'orteil (11) du membre d'avant-pied (16) en au moins deux parties :
- un membre de talon (22) s'étendant de l'unité de fixation (14) à travers la région de cheville (12) vers la région de talon (13), ledit membre de talon (22) comprenant au moins un ressort à lames de talon curvilinéaire allongé (23) ;
- un membre de pied inférieur (25) fixé audit membre de talon (22) dans la région de talon (13), **caractérisé en ce que**
le membre de pied inférieur (25) est fixé au membre d'avant-pied (16) au niveau de ladite région d'orteil (11), et **en ce que**
le membre de pied inférieur (25) est latéralement unitaire le long de sa longueur longitudinale
et **en ce que** l'au moins une rainure de séparation (20) a une longueur s'étendant d'une région d'orteil antérieur (21) vers l'unité de fixation (14) le long de seulement 15 à 50 % de la longueur longitudinale du membre d'avant-pied (16).

2. Dispositif de pied prothétique (10) selon la revendication 1, dans lequel l'au moins une rainure de séparation (20) a une longueur s'étendant d'une région d'orteil antérieure (21) vers l'unité de fixation (14) le long de seulement 25 à 35 %, plus préférablement le long de seulement 30 % de la longueur longitudinale du membre d'avant-pied (16).

3. Dispositif de pied prothétique (10) selon les revendications 1 et 2, dans lequel le membre de pied inférieur (25) est relié au membre d'avant-pied (16) et au membre de talon (22) par un adhésif élastomère ayant un module de traction (E) de moins de 13 MPa.

4. Dispositif de pied prothétique (10) selon la revendication 3, dans lequel l'adhésif élastomère est appliqué sur des premières zones de liaison (28) entre le membre d'avant-pied (16) et le membre de pied inférieur (25) dans la région d'orteil (11), lesdites premières zones de liaison (28) s'étendant dans la direction longitudinale de la région d'orteil antérieur (11) le long de 25 à 60 %, plus préférablement le long de 30 à 55 %, le plus préférablement 40 à 50 % de la longueur de l'au moins une rainure de séparation (20) du membre d'avant-pied (16) et vers la région de cheville (12), et 30 à 60 %, de préférence 40 à 55 %, et plus préférablement 50 % de la largeur dans les directions latérales à partir de ladite rainure de séparation (20).

5. Dispositif de pied prothétique (10) selon les revendications 2 à 4, dans lequel l'adhésif élastomère est appliqué sur une deuxième zone de liaison (29) entre le membre d'avant-pied (16) et le membre de pied inférieur (25) dans la région de talon (13).

6. Dispositif de pied prothétique (10) selon l'une quelconque des revendications précédentes, dans lequel le membre d'avant-pied (16) comprend au moins deux ressorts à lames d'avant-pied curvilinéaires allongés (17) étant reliés dans la région d'orteil (11) et formant des arcs fourchus et concaves vers l'avant définissant des premiers espaces (18) entre les au moins deux ressorts à lames d'avant-pied (17) dans la région de cheville (12).

7. Dispositif de pied prothétique (10) selon l'une quelconque des revendications précédentes, dans lequel le membre d'avant-pied (16) comprend deux ressorts à lames d'avant-pied curvilinéaires allongés (17) étant reliés dans la région d'orteil (11) et formant des arcs bifurqués concaves vers l'avant définissant un premier espace (18) entre lesdits ressorts à lames d'avant-pied (17) dans la région de cheville (12).

8. Dispositif de pied prothétique (10) selon l'une quelconque des revendications 6 et 7, dans lequel les ressorts à lames d'avant-pied (17) du membre d'avant-pied supérieur (16) sont reliés par un adhésif élastomère appliqué sur des troisièmes zones de liaison (30), lesdites troisièmes zones de liaison (30) s'étendant dans la direction longitudinale depuis la région d'orteil antérieure (11) le long de 25 à 60 %, plus préférablement le long de 35 à 55 %, le plus préférablement le long de 40 à 50 % de la longueur de l'au moins une rainure de séparation (20) des ressorts à lames d'avant-pied (17) et vers la région de cheville (12), et au moins 50 %, de préférence au moins 75 %, et plus préférablement au moins 90 % de la longueur dans les directions latérales de ladite rainure de séparation (20).

9. Dispositif de pied prothétique (10) selon l'une quelconque des revendications 1 à 8, dans lequel le membre de talon (22) comprend au moins deux ressorts à lames de talon curvilinéaires allongés (23) s'étendant à partir de l'unité de fixation (14) vers le bas et vers l'avant en direction de la région d'orteil, tournant autour de la région de cheville (12) et vers l'arrière en direction de la région de talon (13) définissant des deuxièmes espaces (24) entre les au moins deux ressorts à lames de talon (23) dans la région de cheville (12).

10. Dispositif de pied prothétique (10) selon l'une quelconque des revendications 1 à 9, dans lequel le membre de talon (22) comprend deux ressorts à lames de talon curvilinéaires allongés (23) s'étendant à partir de l'unité de fixation (14) vers le bas et vers l'avant en direction de la région d'orteil, tournant autour de la région de cheville (12) et vers l'arrière en direction de la région de talon (13) définissant un deuxième espace (24) entre les deux ressorts à lames de talon (23) dans la région de cheville (12).

11. Dispositif de pied prothétique (10) selon l'une quelconque des revendications 9 et 10, dans lequel les ressorts à lames de talon (23) sont reliés par un adhésif élastomère appliqué sur une quatrième zone de liaison (31).

12. Dispositif de pied prothétique (10) selon l'une quelconque des revendications précédentes 4 à 11, dans lequel l'adhésif élastomère appliqué sur le première (28) et deuxième (29) zones de liaison est un premier adhésif élastomère ayant un module de traction (E) inférieur à un deuxième adhésif élastomère appliqué sur les troisième (30) et quatrième (31) zones de liaison.

13. Dispositif de pied prothétique (10) selon la revendication 12, dans lequel le premier adhésif élastomère a un module de traction (E) inférieur à 5 MPa, plus préférablement inférieur à 3 MPa, le plus préférablement de 1 MPa.

14. Dispositif de pied prothétique (10) selon la revendication 12, dans lequel le deuxième adhésif élastomère a un module de traction (E) de 13 à 7 MPa, plus préférablement de 12 à 8 MPa, le plus préférablement de 10 MPa.

15. Dispositif de pied prothétique (10) selon l'une quelconque des revendications précédentes 1 à 14, dans lequel les ressorts à lames d'avant-pied (17), les ressorts à lames de talon (23) et le membre de pied inférieur (25) sont faits d'un matériau flexible et résistant.
